# Europäisches Patentamt
## European Patent Office
## Office européen des brevets

(11) Veröffentlichungsnummer: **0 031 113**
**A1**

(12) **EUROPÄISCHE PATENTANMELDUNG**

(21) Anmeldenummer. **80107893.2**

(22) Anmeldetag: **13.12.80**

(51) Int. Cl.³: **C 07 C 45/33**
C 07 C 29/50, C 07 C 49/403
C 07 C 35/08, C 07 C 179/03

(30) Priorität: **22.12.79 DE 2951956**

(43) Veröffentlichungstag der Anmeldung·
**01.07.81 Patentblatt 81/26**

(84) Benannte Vertragsstaaten.
**BE CH DE FR GB IT LI NL**

(71) Anmelder: **BASF Aktiengesellschaft**
**Carl-Bosch-Strasse 38**
**D-6700 Ludwigshafen(DE)**

(72) Erfinder **Rieber, Norbert, Dr.**
**Liebfrauenstrasse 1C**
**D-6800 Mannheim(DE)**

(72) Erfinder: **Platz, Rolf, Dr.**
**Hansastrasse 5**
**D-6800 Mannheim(DE)**

(72) Erfinder: **Fuchs, Werner, Dr.**
**Muenchbuschweg 30B**
**D-6700 Ludwigshafen(DE)**

(72) Erfinder: **Stabenow, Joachim, Dr.**
**Am Feldrain 22**
**D-6940 Weinheim(DE)**

(72) Erfinder: **Herrmann, Guenter, Dr.**
**Haeuserstrasse 21**
**D-6900 Heidelberg(DE)**

(72) Erfinder: **Wilfinger, Hans Joerg, Dr.**
**Bitzstrasse 15B**
**D-6707 Schifferstadt(DE)**

(72) Erfinder: **Hellbach, Hans**
**Stettiner Strasse 14**
**D-6840 Lampertheim(DE)**

(54) **Verfahren zur Herstellung von Gemischen aus Cyclohexylhydroperoxid, Cyclohexanon und Cyclohexanol.**

(57) Verfahren zur kontinuierlichen Herstellung von Gemischen aus Cyclohexylhydroperoxid, Cyclohexanol und Cyclohexanon mit einem hohen Gehalt an Peroxiden durch Umsetzen von Cyclohexan mit molekularem Sauerstoff in flüssiger Phase bei einer Temperatur von 160 bis 210°C und unter einen Druck von 12 bis 60 bar in mehreren hintereinander geschalteten Stufen, wobei man die Temperatur von Stufe zu Stufe um jeweils mindestens 5°C senkt und in jeder Stufe eine Verweilzeit von 1,8 bis 5 Minuten enthält.

BASF Aktiengesellschaft                    O.Z. 0050/034206

Verfahren zur Herstellung von Gemischen aus Cyclohexylhydroperoxid, Cyclohexanon und Cyclohexanol

Um die Ausbeute an Cyclohexanol und Cyclohexanon zu verbessern, wird bei der Oxidation von Cyclohexan zu Cyclohexanol und Cyclohexanon zweistufig verfahren. Zunächst wird
Cyclohexan mit molekularem Sauerstoff enthaltenden Gasen
zu einem Gemisch aus Cyclohexylhydroperoxid, Cyclohexanon
und Cyclohexanol oxidiert und anschließend Cyclohexylhydroperoxid katalytisch zu Cyclohexanol und Cyclohexanon
zersetzt. Ein solches Verfahren ist beispielsweise aus der
DE-OS 17 68 529 und aus der DAS 19 17 814 bekannt. Dabei
wird Cyclohexan mit Luft zu einem Cyclohexylhydroperoxid
enthaltenden Reaktionsgemisch oxidiert. Da Metallionen,
die aus den Reaktorwänden stammen, Peroxide zersetzen, ist
es jedoch erforderlich, die Innenwandungen mit Natriumpyrophosphat zu passivieren. Dies ist aufwendig und die Passivierung ist gegen Beschädigungen empfindlich. Darüber
hinaus ergibt sich durch die Verwendung von Gasen mit
einem niedrigen Sauerstoffgehalt der Nachteil, daß die
abzuführenden Inerte in aufwendiger Weise von Cyclohexan
befreit werden müssen. Entsprechend der CH-PS 5 10 599
wird die Oxidation von Cyclohexan zu Cyclohexylhydroperoxid mittels reinem Sauerstoff durchgeführt. Hierbei ist
es jedoch erforderlich, den Umsatz auf unter 2 Gew.% zu
beschränken, um eine genügend hohe Ausbeute an Cyclohexylhydroperoxid zu erzielen. Der geringe Umsatz erfordert
jedoch die Umwälzung an erheblichen Mengen an Cyclohexan.

Es war deshalb die technische Aufgabe gestellt, bei der
Oxidation von Cyclohexan zu Cyclohexylhydroperoxid die aufwendige Passivierung von Reaktorinnenwänden zu vermeiden
und darüber hinaus unter Verwendung von reinem Sauerstoff
höhere Umsätze als bisher zu erzielen.

Diese Aufgabe wird gelöst in einem kontinuierlichen Verfahren zur Herstellung von Gemischen aus Cyclohexylhydroperoxid, Cyclohexanol und Cyclohexanon mit einem hohen Gehalt an Peroxiden durch Umsetzen von Cyclohexan mit molekularem Sauerstoff in flüssiger Phase bei einer Temperatur von 160 bis 210°C und unter einem Druck von 12 bis 60 bar in mehreren hintereinander geschalteten Stufen, wobei man die Temperatur von Stufe zu Stufe um jeweils mindestens 5°C senkt und in jeder Stufe eine Verweilzeit 1,8 bis 5 Minuten einhält.

Das neue Verfahren hat den Vorteil, daß man hohe Umsätze erzielt und zugleich einen hohen Gehalt an Cyclohexylhydroperoxid erhält. Ferner hat das neue Verfahren den Vorteil, daß die aufwendige Abgasreinigung wegfällt und zudem ein geringerer apparativer Aufwand erforderlich ist. Darüber hinaus bedarf es keiner Passivierung der Innenflächen der Vorrichtungen oder anderer Zusätze, um Metallionen die Cyclohexylhydroperoxid vorzeitig zersetzen, zu vermeiden.

Erfindungsgemäß wird Cyclohexan mit molekularem Sauerstoff, d.h. reinem Sauerstoff umgesetzt. Der verwendete technische Sauerstoff kann bis zu 10 Gew.% Inerte wie Stickstoff oder Argon enthalten. Die Umsetzung erfolgt bei einer Temperatur von 160 bis 210°C und unter einem Druck von 12 bis 60 bar in flüssiger Phase. Die Umsetzung wird kontinuierlich in mehreren hintereinander geschalteten Stufen durchgeführt. Vorteilhaft wendet man 2 bis 4 Stufen an. Als Reaktoren können Rührkessel Blasensäulen oder andere Reaktortypen eingesetzt werden. Hierbei hat es sich bewährt, jede Stufe geflutet zu betreiben, d.h. daß jede Stufe vollständig mit Reaktionsmedium gefüllt ist und sich keine Gasphase ausbildet. Vorteilhaft wird in jeder Stufe die zugeführte Menge an molekularem Sauerstoff mindestens zu 99% umgesetzt.

0031113

Erfindungsgemäß wird die Temperatur von Stufe zu Stufe um jeweils mindestens 5°C gesenkt. Vorteilhaft senkt man die Temperatur von Stufe zu Stufe jeweils um 5 bis 10°C.

Ein weiteres erdingungswesentliches Merkmal ist es, daß man in jeder Stufe eine Verweilzeit von 1,8 bis 5 Min. einhält. Besonders bewährt haben sich Verweilzeiten von 1,8 bis 3,5 Min.

Die Umsetzung führt man zweckmäßig ohne Zusatz von Katalysatoren durch, um eine Zersetzung des Cyclohexylhydroperoxids zu vermeiden.

Durch Einhaltung der oben aufgeführten Reaktionsbedingungen überziehen sich die Oberflächen der bei der technischen Cyclohexanoxidation üblicherweise eingesetzten Vorrichtungen aus nichtrostendem Stahl während der Oxidation mit einer gleichmäßigen dünnen Passivierungsschicht aus Eisenoxid, die sich durch röntgeographische Analysen und Untersuchungen mit einer Elektronenstrahlmikrosonde nachweisen lassen. Die Passivierung der Metalloberflächen erfolgt in kurzer Zeit. So überzieht sich z.B. ein Probestreifen aus nickelhaltigem rostfreiem Stahl (Werkstoff-Nr. nach DIN 1.4541 und 1.4571) bereits nach wenigen Stunden vollständig mit einer gleichmäßig fest haftenden passivierenden Eisenoxidschicht.

Das erhaltene Reaktionsgemisch enthält neben nichtumgesetztem Cyclohexan Cyclohexylhydroperoxid, Cyclohexanol, Cyclohexanon und geringe Mengen anderer sauerstoffhaltiger Oxidationsprodukte wie Ester und Säuren. Bezogen auf das umgesetzte Cyclohexan erhält man Cyclohexylhydroperoxid in Mengen bis zu über 70 Gew.%.

Aus dem erhaltenen Reaktionsgemisch werden vor der weiteren Verarbeitung vorteilhaft ein Teil der sauren Bestandteile durch Wasser ausgewaschen. Die weitere Umsetzung zu Cyclohexanol und Cyclohexanon erfolgt beispielsweise wie in der DE-OS 17 68 529 beschrieben. Cyclohexanon und Cyclohexanol dienen zur Herstellung von Adipinsäure und Caprolactam.

Das Verfahren nach der Erfindung sei an folgenden Beispielen beschrieben.

### Beispiel 1

Man verwendet zwei hintereinander geschaltete Hochdruckgefäße aus nichtrostendem Stahl von jeweils 20 l Inhalt. Beide Hochdruckgefäße sind mit Cyclohexan vollständig gefüllt. In das erste Hochdruckgefäß leitet man stündlich 500 l auf 190 bis 200°C erhitztes Cyclohexan über die Innenbohrung einer Zweistoffdüse ein. Über den außenliegenden Ringspalt der Zweistoffdüse führt man stündlich 2400 N l Sauerstoff zu. Im ersten Hochdruckgefäß hält man eine Temperatur von 193 bis 198°C und einen Druck von 14 bis 17 bar ein. Das erhaltene Reaktionsgemisch wird dann durch einen Kühler über die Innenbohrung der Zweistoffdüse dem zweiten Hochdruckgefäß zugeführt und dort ebenfalls stündlich 2400 N l Sauerstoff zugeführt. In der zweiten Stufe hält man eine Temperatur von 180 bis 190°C und einen Druck von 14 bis 17 bar ein. Die Verweilzeit in jedem Hochdruckgefäß beträgt 2,4 Min. Im so erhaltenen Reaktionsgemisch sind 4,9 Gew.% Cyclohexan umgesetzt und der Hydroperoxidgehalt beträgt entsprechend titrimetischer Messung 3,5 Gew.%.

Beispiel 2

Man verfährt wie in Beispiel 1 beschrieben, verwendet jedoch drei Hochdruckgefäße von jeweils 20 l Rauminhalt. Unter sonst gleichen Bedingungen erhält man im ersten Hochdruckgefäß eine Temperatur von 190 bis 195°C, im zweiten Hochdruckgefäß eine Temperatur von 180 bis 187°C und im dritten Hochdruckgefäß eine Temperatur von 170 bis 177°C ein. Es versteht sich, daß in das dritte Hochdruckgefäß ebenfalls die gleiche Menge an Sauerstoff zugeführt wird wie in den ersten beiden Hochdruckgefäßen ca. 1500 Nl $O_2$ je Reaktor. Der Cyclohexanumsatz beträgt 4,7 % und die Hydroperoxidkonzentration 3,1 Gew.%.

Vergleichsbeispiel 1

Verfahren wird wie in Beispiel 2. Die Cyclohexan-Zufuhr liegt bei 720 l/Stunde, die Sauerstoffmenge pro Reaktor bei 2,5 Nm³/Stunde, die Verweilzeit pro Reaktor bei 1,66 Min. und die Reaktionstemperatur bei 194 bis 198°C im ersten, bei 175 bis 180°C im zweiten und bei 167 bis 175°C im dritten. Reaktor.

Im so erhaltenen Reaktionsgemisch sind 4,9 Gew.% Cyclohexan umgesetzt und der Hydroperoxydgehalt beträgt nur 2,4 Gew.%.

Vergleichsbeispiel 2

Man verwendet zwei hintereinander geschaltete Hochdruckgefäße aus nichtrostendem Stahl von jeweils 1 l Inhalt. Beide Druckgefäße sind mit Cyclohexan vollständig gefüllt. In das erste Druckgefäß leitet man stündlich 20 l auf 190°C erhitztes Cyclohexan über die Innenbohrung einer Zweistoffdüse ein. Über den außenliegenden Ringspalt der Zweistoffdüse führt man stündlich 150 Nl Sauerstoff zu. In

beiden Hochdruckgefäßen hält man eine Temperatur von 191 bis 193°C und einen Druck von 14 bis 16 bar ein. Das Reaktionsgemisch des ersten Reaktors wird über die Innenbohrung einer Zweistoffdüse dem zweiten Reaktor zugeführt und dort ebenfalls stündlich 150 Nl Sauerstoff eingeleitet. Die Verweilzeit in jedem Reaktor beträgt 3 Min.

Im so erhaltenen Reaktionsgemisch sind 5,1 Gew.% Cyclohexan umgesetzt und der Hydroperoxydgehalt beträgt nur 2,4 Gew.%.

Vergleichsbeispiel 3

A. Man verwendet zwei hintereinander geschaltete Rührautoklaven aus nichtrostendem Stahl von jeweils 1 l Inhalt. Beide Autoklaven sind mit Cyclohexan vollständig gefüllt. In den ersten Autoklaven leitet man stündlich bei einer Rührerdrehzahl von 1000 UPM 10 l auf 190°C erhitztes Cyclohexan ein. Über ein Einsteckrohr führt man stündlich 75 Nl Sauerstoff zu. In beiden Rührautoklaven hält man eine Temperatur von 190°C und einen Druck von 14 bis 16 bar ein. Das Reaktionsgemisch des ersten Autoklaven wird über ein Einsteckrohr dem zweiten Autoklaven zugeführt und dort stündlich ebenfalls 75 Nl Sauerstoff bei einer Rohrerdrehzahl von 1000 UPM eingeleitet. In jedem Autoklaven beträgt die Verweilzeit 6 Min.

Im so erhaltenen Reaktionsgemisch sind 5,3 Gew.% Cyclohexan umgesetzt und der Hydroperoxydgehalt beträgt 1,9 Gew.%.

B.   Es wird verfahren wie bei A.

Die Cyclohexanzufuhr liegt bei 20 l/Std., die Sauerstoffmenge pro Reaktor bei 150 Nl/Std. und die Verweilzeit pro Reaktor bei 3 Min. Im so erhaltenen
Reaktionsgemisch sind 5,2 Gew.% Cyclohexan umgesetzt
und der Hydroperoxydgehalt beträgt 2,2 Gew.%.

Patentansprüche

1.  Verfahren zur kontinuierlichen Herstellung von Gemischen aus Cyclohexylhydroperoxid, Cyclohexanol und Cyclohexanon mit einem hohen Gehalt an Peroxiden durch Umsetzen von Cyclohexan mit molekularem Sauerstoff in flüssiger Phase bei einer Temperatur von 160 bis 210$^{o}$C und unter einen Druck von 12 bis 60 bar in mehreren hintereinander geschalteten Stufen, dadurch gekennzeichnet, daß man die Temperatur von Stufe zu Stufe um jeweils mindestens 5$^{o}$C senkt und in jeder Stufe eine Verweilzeit von 1,8 bis 5 Min. einhält.

2.  Verfahren nach Anspruch 1, dadurch gekennzeichnet, daß man die Temperatur von Stufe zu Stufe um jeweils 5 bis 10$^{o}$C senkt.

3.  Verfahren nach den Ansprüchen 1 und 2, dadurch gekennzeichnet, daß man in jeder Stufe eine Verweilzeit von 1,8 bis 3,5 Min. einhält.

4.  Verfahren nach den Ansprüchen 1 bis 3, dadurch gekennzeichnet, daß die Reaktionsstufen vollständig geflutet sind.

5.  Verfahren nach den Ansprüchen 1 bis 4, dadurch gekennzeichnet, daß man die in jeder Stufe zugeführte Sauerstoffmenge mindestens zu 99% umsetzt.

# EUROPÄISCHER RECHERCHENBERICHT

**Europäisches Patentamt**

Nummer der Anmeldung
EP 80 10 7893

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | FR - A - 2 291 956 (ICI) <br> * Ansprüche 1-6,9,10; Seite 3, Zeilen 34-37; Seite 4, Zeilen 25-28; Seite 5, Zeilen 12-20; Seite 5, Zeile 34 bis Seite 6, Zeile 8 * <br><br> & DE - A - 2 252 119 <br> -- | 1,3 |
| | DE - A - 2 136 744 (E.I. DU PONT DE NEMOURS) <br> * Anspruch 1; Seite 4, Absatz 2; Beispiel; Seite 10 * <br> -- | 1,2 |
| A | CH - A - 406 181 (HALCON INTERNATIONAL) <br> * Seite 1, Zeilen 38-49; Anspruch * <br><br> & DE - A - 1 468 572 <br> -- | 1 |
| A | FR - A - 2 106 768 (FURMAN MENDEL SIMKHOVICH) <br> * Anspruch 1; Seite 2, Zeilen 36-38 * <br> ----- | 1,3 |

### KLASSIFIKATION DER ANMELDUNG (Int. Cl.³)

C 07 C 45/33
29/50
49/403
35/08
179/03

### RECHERCHIERTE SACHGEBIETE (Int. Cl.³)

C 07 C 45/33
29/50
49/403
35/08
179/03

### KATEGORIE DER GENANNTEN DOKUMENTE

X. von besonderer Bedeutung
A technologischer Hintergrund
O nichtschriftliche Offenbarung
P Zwischenliteratur
T der Erfindung zugrunde liegende Theorien oder Grundsätze
E kollidierende Anmeldung
D in der Anmeldung angeführtes Dokument
L aus andern Gründen angeführtes Dokument
& Mitglied der gleichen Patentfamilie, übereinstimmendes Dokument

Der vorliegende Recherchenbericht wurde für alle Patentansprüche erstellt

| Recherchenort | Abschlußdatum der Recherche | Prüfer |
|---|---|---|
| Den Haag | 04-03-1981 | BONNEVALLE |

EPA form 1503.1 06.78